# FASCICULE DE BREVET EUROPEEN

(11) **EP 1 762 675 B1**
(45) Date de publication et mention de la délivrance du brevet: **11.11.2009**
(21) Numéro de dépôt: 06358015.3
(22) Date de dépôt: 13.09.2006
(51) Int. Cl.: E04H 13/00, B01D 47/02, B01D 53/14

(54) **Dispositif d'épuration de l'air pour caveaux mortuaires ou enfeus étanches**
Luftreinigungsvorrichtung für Grabgewölben oder dichten Grabnischen
Air purification device for burial vaults or sealed wall-niche tombs

(30) Priorité: 13.09.2005 FR 0509347
(43) Date de publication de la demande: 14.03.2007
(73) Titulaire: Belliardo-Lusso, Jacqueline, 83210 Sollies-Toucas (FR); Guyetand, Didier, 83210 Sollies-Toucas (FR)
(72) Inventeur: Belliardo-Lusso, Jacqueline, 83210 Sollies-Toucas (FR); Guyetand, Didier, 83210 Sollies-Toucas (FR)
(74) Mandataire: Roman, Michel

(56) Documents cités:
- FR-A- 2 837 721
- US-A- 2 665 471

## Description

La présente invention a pour objet un dispositif d'épuration de l'air pour caveaux mortuaires ou enfeus étanches.

Il est destiné à la filtration et à la purification des gaz s'échappant, lors de la décomposition des corps, des cellules mortuaires étanches telles que caveaux à une ou plusieurs places et enfeus simples ou en batteries, tout en permettant la pénétration de l'air extérieur.

La plupart des caveaux ou enfeus réalisés à ce jour sont soit étanches, soit pourvus d'un simple filtre à air ou d'un système à siphon, simple ou double. Si ces systèmes sont efficaces pour arrêter les odeurs et les micro-organismes tels que les moisissures participant à la décomposition du corps, ils n'ont en revanche aucun effet contre les microbes, virus et similaires.

Le brevet N° FR 2 547 609 déposé par M. Francis AUGIAS fait état d'un dispositif équilibreur d'aération de caveaux caractérisé par la combinaison d'une chambre d'admission et d'une chambre d'évacuation contenant toutes deux un fluide constituant un clapet anti-retour.

Un autre dépôt du même inventeur, publié sous le N° FR 2 461 795, décrit une cellule mortuaire à double ventilation extérieure et intérieure par appareil purificateur de gaz avec admission d'air forcée et échappement sous faible pression.

Le brevet N° FR 2 651 522, toujours du même inventeur, concerne un système de filtration pour caveaux mortuaires constitué par la combinaison de deux enceintes, disposées à l'intérieur de la cellule mortuaire, traversées successivement par l'air sortant du caveau ou enfeu, la première contenant une masse filtrante absorbante, à base de charbon actif par exemple, la seconde étant remplie d'un matériau oxydant détruisant les microbes et virus, l'air purifié aboutissant à l'extérieur grâce à une cheminée pourvue d'une grille anti-insectes et communiquant avec la seconde enceinte.
Ces dispositifs, en raison de leur complexité (double chambre, air forcé, etc), sont d'un prix de revient élevé et ne sont pas agencés pour désodoriser les gaz issus des cellules mortuaires.

Le brevet N° FR 2 837 721 A déposé par les auteurs de la présente demande décrit un filtre d'épuration constitué par la combinaison d'un conduit d'admission d'air à clapet anti-retour et d'un tube translucide raccordé à un conduit d'évacuation et contenant un liquide désodorisant et désinfectant traversé par les gaz de la cellule mortuaire, sous l'effet de la surpression naturelle, grâce à un plongeur à filtre minéral débouchant dans ledit liquide.
Cet appareil monobloc, conçu pour être monté entre deux murs, est encombrant et doit être adapté à chaque application. Il est assez difficile à installer, car il nécessite deux perçages se faisant face et situés dont la position doit être précise. Il nécessite en outre un démontage pour le remplacement du liquide de filtration, ce qui rend l'opération longue et coûteuse.

Le dispositif connu du document FR 2 837 721 A présente les caractéristiques du préambule de la revendication 1.

Le brevet N° US 2 665 471 déposé par M. GOULE décrit un dispositif d'épuration de l'air pour cellules mortuaires étanches comprenant un module d'entrée d'air avec clapet d'aspiration anti-retour et un module filtrant physiquement indépendants. Cependant, il est difficile de changer le liquide filtrant dans ce type de dispositif.

Le dispositif selon la présente invention a pour objectif de remédier à ces inconvénients. Il permet en effet de réaliser des appareils de filtration simples, peu encombrants et de faible coût, s'adaptant à tous les types de caveaux avec sorties frontales, latérales ou perpendiculaires et dont l'installation et l'entretien sont sensiblement facilités par rapport aux appareils existants.

Il est constitué de deux éléments physiquement indépendants : un module d'entrée d'air avec clapet d'aspiration anti-retour, et un module filtrant contenant un liquide de désodorisation et désinfection traversé par les gaz de la cellule mortuaire. Sous l'effet de la surpression naturelle, les gaz de décomposition de la cellule mortuaire devront obligatoirement passer, pour ressortit épurés, à travers ce liquide, qui possède également un neutralisant. Conformément à l'invention, le liquide est contenu dans une cartouche interchangeable. Cette caractéristique technique permet de remplacer très facilement le liquide filtrant lors de chaque inhumation, sans danger pour l'environnement, par une nouvelle cartouche liquide, sans changement de l'appareil.

Sur les dessins schématiques annexés, donnés à titre d'exemples non limitatifs de formes de réalisation de l'objet de l'invention :
- la figure 1 est une vue latérale en élévation d'un module de filtration monté dans la paroi d'un caveau mortuaire,
- la figure 2 représente dans les mêmes conditions un module d'entrée d'air,
- les figures 3 et 4 montrent dans les mêmes conditions une variante avec conduits de passage des gaz prolongés verticalement vers le haut
- la figure 5 est une vue latérale en élévation d'un module de filtration monté dans la paroi d'un caveau mortuaire par l'intermédiaire d'un moyen de fixation dans une variante de réalisation,
- la figure 6 est une vue en perspective du moyen de fixation représenté sur la figure 5,
- la figure 7 est une vue latérale en élévation d'un module de filtration monté dans la paroi d'un caveau mortuaire par l'intermédiaire d'un moyen de fixation dans une autre variante de réalisation.

Le dispositif est constitué d'un module filtrant 1 contenant un liquide épurateur et comportant un conduit d'évacuation 2, et d'un module d'entrée d'air 3 formé d'un conduit d'admission 4 de l'air extérieur. Les conduits d'admission et d'évacuation comportent des extrémités filetées 5, 5' destinées à traverser les parois 6 du caveau et à recevoir un bouchon-grille 15 contre les insectes, et un opercule en caoutchouc pour le transport.
Les deux modules sont séparés pour faciliter la mise en place du dispositif et son adaptation à des caveaux de dimensions différentes.

Le module de filtration 1 est rempli d'un liquide de filtration désodorisant et désinfectant non volatile conservant ses propriétés dans le temps. En pratique, on utilise préférentiellement un liquide de filtration BIOLOR®, tout autre liquide de filtration connu de l'homme du métier pouvant être employé.
Le liquide de filtration, remplaçable, est contenu dans un élément tubulaire 7 équipé d'un plongeur monté en partie basse du module et obturé par un bouchon 8 en en PVC.
Ce liquide, qui possède également un neutralisant, pourra être remplacé très facilement, par une nouvelle cartouche liquide lors de chaque inhumation, sans changement de l'appareil et sans danger pour l'environnement.
Le neutralisant et la recharge (cartouche de liquide de remplacement) pourront être livrés, à la demande, avec l'appareil.

A la partie haute du module de filtration 1, se trouve une ouverture 9 débouchant dans la cellule mortuaire et munie d'une grille pare-insectes et d'un bouchon de protection 10 pour le stockage et le transport du dispositif. Lors de la putréfaction des corps, il se crée une surpression qui chassera les gaz de décomposition qui ne pourront s'échapper que par cette ouverture et devront obligatoirement emprunter le circuit de filtration.
Les gaz vont alors barboter dans le liquide de filtration pour ressortir épurés à l'extérieur de la cellule par l'intermédiaire du conduit d'évacuation 2.

Le module d'entrée d'air 3 est équipé d'un clapet anti-retour d'un modèle hypersensible, pour faciliter la combustion du corps.

Les conduits d'admission d'air 4 et d'évacuation 2 de gaz filtré seront avantageusement réalisés en tubes PVC-pression d'environ 50 mm de diamètre.

Dans le cas des cimetières paysagés par exemple, des sorties spéciales 11 dans la pelouse ou tout autre environnement sont également prévues à cet effet. Le conduit d'évacuation 2, et/ou le conduit d'admission 4 seront équipés de prolongations verticales 12 pourvues d'un chapeau de ventilation et raccordées par des coudes 13 aux dits conduits.

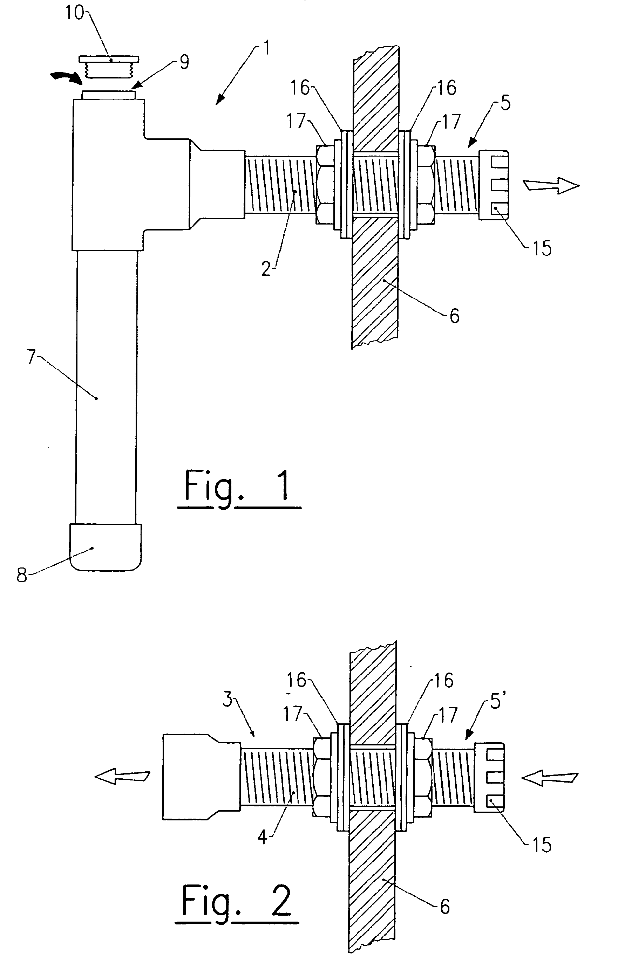

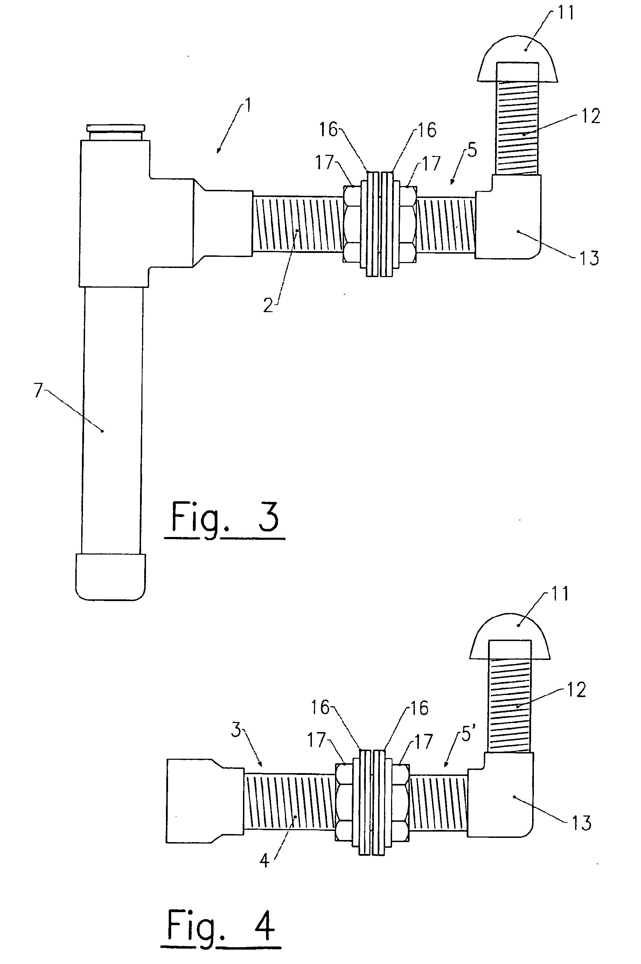

En se rapportant aux modes de réalisation représentés sur les figures 1 à 4, les conduits d'admission d'air 4 et d'évacuation 2 de gaz filtré sont filetés et maintenus en position sur les parois 6 du caveau par l'intermédiaire d'un dispositif de fixation classique constitué de rondelles 16 et d'écrous 17 visés sur lesdits conduits. On utilise avantageusement un empilement de rondelles 16 en PVC et en caoutchouc de manière à assurer un maintien en position efficace.

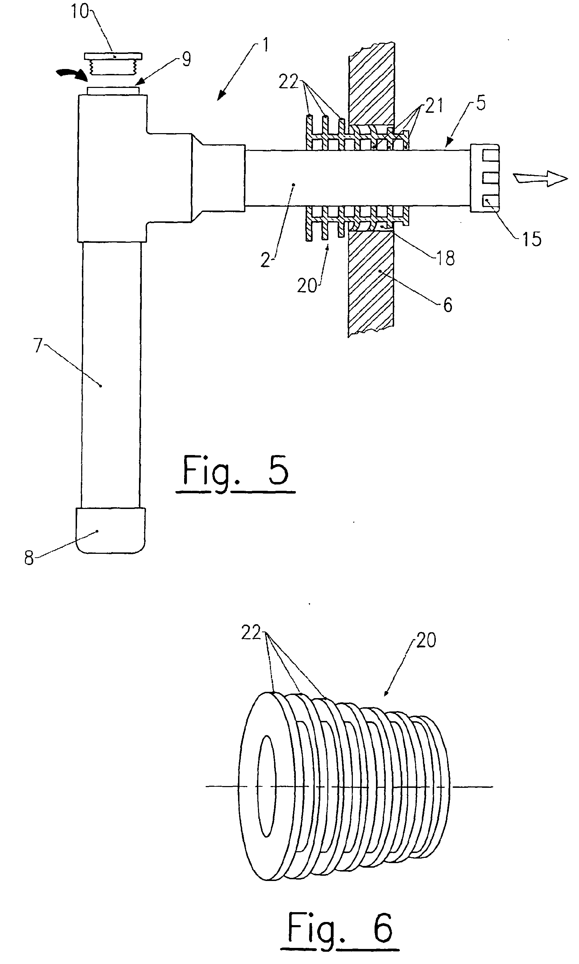

Dans une variante de réalisation représentée sur les figures 5 à 7, le conduit d'évacuation 2 est équipé d'un manchon 20 destiné à se positionner dans un alésage 8 prévu sur la paroi 6 du caveau, ledit manchon étant configuré de manière à rentrer en force dans ledit alésage et comportant des moyens aptes à se déformer pour assurer une étanchéité à l'air.

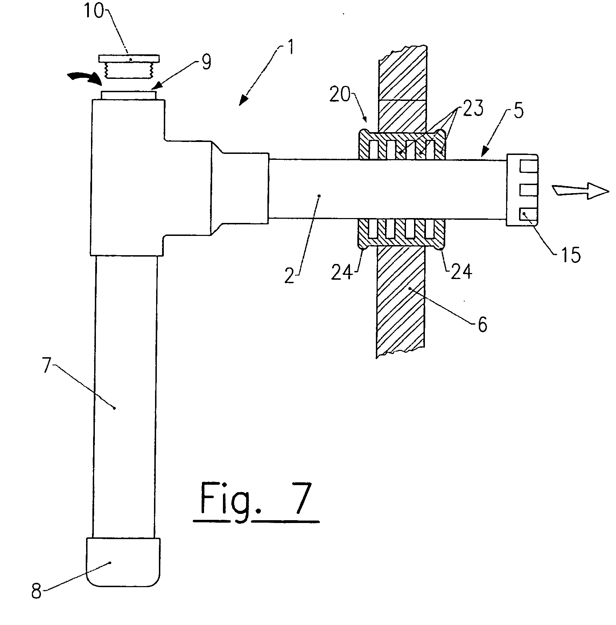

Les figures 5 et 7 schématisent uniquement le maintien en position du conduit d'évacuation 2, mais le même manchon est utilisable pour le conduit d'admission d'air 4.
L'utilisation d'un tel manchon permet de monter plus facilement et plus rapidement les conduits 2 et 4 et évite d'avoir à employer des conduits filetés onéreux.

Selon un premier mode de réalisation représenté sur les figures 5 et 6, des lèvres intérieures 21 radiales sont agencées sur le surface intérieure du manchon 20 de sorte que lorsque le conduit d'évacuation 2 - ou le conduit d'admission 4 - se positionne dans ledit manchon, lesdites lèvres forment étanchéité à l'air entre ledit conduit et ledit manchon. En pratique les lèvres 21 sont réalisées dans un matériau souple et déformable et sont dimensionnées de manière à ce que le diamètre intérieur du manchon 20 soit inférieur au diamètre extérieur du conduit 2 - ou du conduit 4. Ainsi, lorsque le conduit d'évacuation 2 - ou d'admission 4 - est mis en place, les lèvres 21 exercent une pression sur ledit conduit et forment étanchéité à l'air.

Le manchon 20 comporte des lèvres extérieures 22 radiales configurées de manière à former étanchéité à l'air entre ledit manchon et l'alésage 18. Ces lèvres 22 ont avantageusement une hauteur décroissante de manière à donner au manchon 20 une forme générale extérieure conique (figure 6). Le manchon 20 a un diamètre extérieur moyen sensiblement inférieur au diamètre de l'alésage 18 de sorte que certaines des lèvres extérieures 22 ont un diamètre supérieur ou égal au diamètre dudit alésage. En pratique les lèvres 22 sont réalisées dans un matériau souple et déformable. Ces caractéristiques techniques impliquent que lorsque le manchon 20 est rentré en force dans l'alésage 18, certaines lèvres extérieures 22 se déforment et forment étanchéité à l'air entre ledit manchon et ledit alésage.
En se référant à la figure 5, les lèvres extérieures 22 sont configurées de manière à se plier dans un sens opposé au sens d'introduction du manchon 20 dans l'alésage. De cette manière, lorsque le manchon 20 est positionné dans l'alésage 18, le pliage des lèvres extérieures 22 assure, en plus de l'étanchéité, le maintien en position dudit manchon.

Selon un second mode de réalisation représenté sur la figure 7, le manchon 20 se présente sous la forme d'un élément cylindrique ayant un diamètre extérieur supérieur ou égal au diamètre de l'alésage 18 de sorte que lorsqu'il est mis en position dans ledit alésage, une étanchéité à l'air soit formée entre ledit manchon et ledit alésage.

De la même façon que décrit précédemment, des lèvres intérieures 23 radiales sont agencées sur la surface intérieure du manchon 20 de sorte que lorsque le conduit d'évacuation 2 ou le conduit d'admission 4 se positionnent dans ledit manchon, lesdites lèvres forment étanchéité à l'air. En pratique les lèvres 23 sont réalisées dans un matériau souple et déformable et sont dimensionnées de manière à ce que le diamètre intérieur du manchon 20 soit inférieur au diamètre extérieur des conduits 2 ou 4. Ainsi, lorsque le conduit d'évacuation 2 ou d'admission 4 est mis en place, les lèvres 23 se déforment et forment étanchéité à l'air entre ledit conduit et le manchon 20.
Des butées 24 sont avantageusement positionnées sur la surface extérieure du manchon 20 de manière à ce que ce dernier soit efficacement maintenu en position sans risque de déboîtement.

En pratique, on préfère employer un manchon 20 obtenu par moulage plastique par injection. Tout autre manchon équivalant connu de l'homme du métier et aboutissant au même résultat peut être employé

Afin de faciliter le conditionnement, le transport et sa mise en oeuvre, le système sera avantageusement entièrement étanche et livré prêt à fonctionner, à mettre en place en l'état, le liquide épurateur du module filtrant 1 étant incorporé.
De préférence, l'appareil sera livré sous la forme d'un conditionnement ("kit") comprenant :
- un module filtrant 1,
- un module d'entré d'air 3,
- une recharge de liquide filtrant en cartouche,
- un neutralisant du liquide filtrant,
- selon le cas, un manchon 20 pour le maintient en position.

Il n'est ainsi plus nécessaire de changer l'épurateur, comme le précise la norme, à chaque inhumation, seul le produit est à changer, le tout dans le respect de l'environnement.

Le positionnement des divers éléments constitutifs donne à l'objet de l'invention un maximum d'effets utiles qui n'avaient pas été, à ce jour, obtenus par des dispositifs similaires.

## Revendications

1. Dispositif d'épuration de l'air pour cellules mortuaires étanches telles que caveaux à une ou plusieurs places et enfeus simples ou en batteries, destiné à la filtration et à la purification des gaz s'échappant, lors de la décomposition des corps, tout en permettant la pénétration de l'air extérieur, et constitué d'un module d'entrée d'air (3) avec clapet d'aspiration anti-retour et d'un module filtrant (1) contenant un liquide désodorisant et désinfectant traversé par les gaz de la cellule mortuaire, **caractérisé en ce que** ledit module d'entrée d'air (3) avec clapet d'aspiration anti-retour et ledit module filtrant (1) sont deux éléments physiquement indépendants, ledit liquide étant contenu dans une cartouche interchangeable.

2. Dispositif d'épuration selon la revendication 1, **se caractérisant par le fait qu'**il est livré sous la forme d'un conditionnement comprenant un module filtrant (1), un module d'entré d'air (3), une recharge de liquide filtrant en cartouche et un neutralisant du liquide filtrant.

3. Dispositif d'épuration selon l'une des revendications précédentes, **se caractérisant par le fait que** le liquide désodorisant et désinfectant est non volatil et est déterminé pour conserver ses propriétés dans le temps.

4. Dispositif d'épuration selon l'une quelconque des revendications précédentes, **se caractérisant par le fait que** le liquide de filtration est contenu dans un élément tubulaire (7) monté en partie basse du module filtrant (1) et obturé par un bouchon (8) en PVC.

5. Dispositif d'épuration selon l'une quelconque des revendications précédentes, **se caractérisant par le fait que** le module filtrant (1) comporte une ouverture (9) débouchant dans la cellule mortuaire pourvue d'une grille pare-insectes et d'un bouchon de protection (10) pour le stockage et le transport du dispositif.

6. Dispositif d'épuration selon l'une quelconque des revendications précédentes, **se caractérisant par le fait que** le clapet anti-retour du module d'entrée d'air (3) est un modèle hypersensible.

7. Dispositif d'épuration selon l'une quelconque des revendications précédentes, **se caractérisant par le fait que** le conduit d'évacuation (2) du module filtrant (1) et le conduit d'admission (4) du module d'entrée d'air (3) comportent des extrémités filetées (5, 5') destinées à traverser les parois (6) du caveau.

8. Dispositif d'épuration selon l'une quelconque des revendications précédentes, **se caractérisant par le fait que** le conduit d'évacuation (2) du module filtrant (1) et le conduit d'admission (4) du module d'entrée d'air (3) sont réalisés en tubes PVC-pression d'environ 50 mm de diamètre.

9. Dispositif d'épuration selon l'une quelconque des revendications précédentes, **se caractérisant par le fait que** le conduit d'évacuation (2), et/ou le conduit d'admission (4) sont équipés de prolongations verticales (12) avec sorties spéciales 11 avec chapeau de ventilation, raccordées par des coudes 13 aux dits conduits.

10. Dispositif d'épuration selon l'une quelconque des revendications précédentes, **se caractérisant par le fait que** le conduit d'évacuation (2) du module filtrant (1) et le conduit d'admission (4) du module d'entrée d'air (3) sont équipés d'un manchon (20) destiné à se positionner dans un alésage (18) prévu sur la paroi (6) du caveau, ledit manchon étant configuré de manière à rentrer en force dans ledit alésage et comportant des moyens aptes à se déformer pour assurer une étanchéité à l'air.

11. Dispositif d'épuration selon la revendication 10, **se caractérisant par le fait que** le manchon (20) comporte des lèvres intérieures (21, 23) radiales configurées de manière à former étanchéité à l'air entre le conduit d'évacuation (2) ou le conduit d'admission (4) et ledit manchon.

12. Dispositif d'épuration selon l'une des revendications 10 ou 11, **se caractérisant par le fait que** le manchon (20) comporte des lèvres extérieures (22) radiales configurées de manière à former étanchéité à l'air entre ledit manchon et l'alésage (18).

13. Dispositif d'épuration selon la revendication 12, **se caractérisant par le fait que** les lèvres extérieures (22) sont configurées de manière à se plieur dans un sens opposé au sens d'introduction du manchon (20) dans l'alésage (18).

## Claims

1. Air purification device for use in sealed burial chambers such as burial vaults for one or more bodies and single or multiple burial recesses, designed to filter and purify escaping gases due to body decomposition while allowing the outside air to penetrate, and consisting of an air intake module (3) with non-return suction valve and a filter module (1) containing a deodorizing and disinfecting liquid through which pass the burial chamber gases, **characterized in that** said air intake module (3) with non-return suction valve and aforementioned filter module (1) are two physically separate elements, the aforementioned liquid being contained in an interchangeable cartridge.

2. Purification device according to claim 1, **characterised in that** it is delivered as a package comprising a filter module (1), an air intake module (3), a refill for the cartridge filter liquid and one neutralizing the filter liquid.

3. Purification device according to one of the above claims, **characterized in that** the deodorizing and disinfecting liquid is non-volatile and is composed in order to retain its properties in time.

4. Purification device according to any of the above claims, **characterised in that** the filter liquid is contained in a tubular element (7) assembled in the bottom part of filter module (1) and sealed by a PVC stopper (8).

5. Purification device according to any of the above claims, **characterised in that** filter module (1) has an opening (9) that emerges in the burial enclosure equipped with an anti-insect grille and a protecting stopper (10) for device storage and transport.

6. Purification device according to any of the above claims, **characterised in that** the non-return valve of air intake module (3) is a hyper-sensitive model.

7. Purification device according to any of the preceding claims, **characterised in that** discharge pipe (2) for filter module (1) and intake pipe (4) of air intake module (3) has threaded ends (5, 5') designed to cross walls (6) of the burial vault.

8. Purification device according to any of the above claims, **characterised in that** pipe (2) of filter module (1) and intake pipe (4) of air intake module (3) are made from PVC-pressure piping of approximately 50 mm diameter.

9. Purification device according to any of the above claims, **characterised in that** flue (2), and/or intake pipe (4) are equipped with vertical extensions (12) with special outlets (11) equipped with a ventilation cap, connected by elbows (13) to the said pipes.

10. Purification device according to any of the above claims, **characterised in that** flue (2) of filter module (1) and inlet pipe (4) of air intake module (3) are equipped with a sleeve (20) designed to take up a position in a drilled hole (18) on wall (6) of the burial vault, the said sleeve being configured so as to penetrate by force the aforementioned drilled hole and comprising means capable of deforming in order to ensure air tightness.

11. Purification device according to claim 10, **characterised in that** sleeve (20) has internal lips (21, 23) radial configured so as to create an air seal between discharge pipe (2) or intake pipe (4) and the aforementioned sleeve.

12. Purification device according to any of claims 10 or 11, **characterised in that** sleeve (20) has radial external lips (22) configured so as to create air tightness between the aforementioned sleeve and drilled hole (18).

13. Purification device according to claim 12, **characterised in that** external lips (22) are configured so as to fold in an opposite direction to the direction of insertion into sleeve (20) of drilled hole (18).

## Patentansprüche

1. Vorrichtung zur Reinigung der Luft für luftdichte Grabstätten wie Grabkammern mit einem oder mehreren Plätzen und einfachen Grabnischen oder Grabnischenverbänden, bestimmt für die Filterung und Reinigung der bei der Verwesung der Körper austretenden Gase, indem das Eindringen der Außenluft ermöglicht wird, bestehend aus einem Modul für die Luftzufuhr (3) mit Ansaugrückschlagventil und einem Filtermodul (1), welches eine geruchsneutralisierende und desinfizierende Flüssigkeit enthält, die von den Gasen der Grabstätte durchströmt wird, **dadurch gekennzeichnet, dass** das besagte Modul für die Luftzufuhr (3) mit Ansaugrückschlagventil und dem besagten Filtermodul (1) zwei physisch voneinander unabhängige Elemente sind, wobei die genannte Flüssigkeit in einer auswechselbaren Patrone enthalten ist.

2. Reinigungsvorrichtung gemäß Patentanspruch 1, **dadurch gekennzeichnet, dass** sie in Form einer Verpackung geliefert wird, die ein Filtermodul (1), ein Modul für die Luftzufuhr (3), eine Nachfüllpatrone mit filtrierender Flüssigkeit und einen Geruchsneutralisierer für die filtrierende Flüssigkeit umfasst.

3. Reinigungsvorrichtung gemäß einem der vorhergehenden Patentansprüche, **dadurch gekennzeichnet, dass** die geruchsneutralisierende und desinfizierende Flüssigkeit nicht volatil und so beschaffen ist, dass sie ihre Eigenschaften über die Zeit beibehält.

4. Reinigungsvorrichtung gemäß einem der vorhergehenden Patentansprüche, **dadurch gekennzeichnet, dass** die filtrierende Flüssigkeit sich in einem röhrenförmigen Element befindet (7), das am unteren Ende des Filtermoduls (1) montiert ist und durch eine Kappe (8) aus PVC verschlossen wird.

5. Reinigungsvorrichtung gemäß einem der vorhergehenden Patentansprüche, **dadurch gekennzeichnet, dass** das Filtermodul (1) eine Öffnung (9) umfasst, welche in die Grabstätte mündet, und die mit einem Insektenschutzgitter und einer Schutzkappe (10) für die Lagerung und den Transport der Vorrichtung versehen ist.

6. Reinigungsvorrichtung gemäß einem der vorhergehenden Patentansprüche, **dadurch gekennzeichnet, dass** das Rückschlagventil des Moduls für die Luftzufuhr (3) eine hochempfindliche Ausführung ist.

7. Reinigungsvorrichtung gemäß einem der vorhergehenden Patentansprüche, **dadurch gekennzeichnet, dass** der Abluftkanal (2) des Filtermoduls (1) und der Zufuhrkanal (4) des Moduls für die Luftzufuhr (3) Gewindeenden (5, 5') besitzen, die dazu bestimmt sind, durch die Grabwände (6) hindurchzuführen.

8. Reinigungsvorrichtung gemäß einem der vorhergehenden Patentansprüche, **dadurch gekennzeichnet, dass** der Abluftkanal (2) des Filtermoduls (1) und der Zufuhrkanal (4) des Moduls für die Luftzufuhr (3) aus PVC-Druckrohren mit circa 50 mm Durchmesser gefertigt sind.

9. Reinigungsvorrichtung gemäß einem der vorhergehenden Patentansprüche, **dadurch gekennzeichnet, dass** der Abluftkanal (2) und/oder der Zufuhrkanal (4) mit vertikalen Verlängerungen (12) mit Spezialausgängen (11) mit Ventilationshaube ausgerüstet und durch Kniestücke (13) mit den besagten Kanälen verbunden sind.

10. Reinigungsvorrichtung gemäß einem der vorhergehenden Patentansprüche, **dadurch gekennzeichnet, dass** der Abluftkanal (2) des Filtermoduls (1) und der Zufuhrkanal (4) des Moduls für die Luftzufuhr (3) mit einer Muffe (20) ausgestattet sind, die dazu bestimmt ist, sich in einem Bohrloch (18) zu positionieren, das in der Grabwand (6) vorgesehen ist, wobei die besagte Muffe so beschaffen ist, dass sie erzwungenermaßen in das besagte Bohrloch eintritt und welche die Eigenschaft besitzt, sich zu verformen, um die Luftundurchlässigkeit zu gewährleisten.

11. Reinigungsvorrichtung gemäß Patentanspruch (10), **dadurch gekennzeichnet, dass** die Muffe (20) innere radiale Dichtlippen (21,23) besitzt, die so beschaffen sind, dass sie die Luftundurchlässigkeit zwischen dem Abluftkanal (2) oder dem Zufuhrkanal (4) und der besagten Muffe gewährleisten.

12. Reinigungsvorrichtung gemäß einem der Patentansprüche 10 oder 11, **dadurch gekennzeichnet, dass** die Muffe (20) äußere radiale Dichtlippen (22) besitzt, die so beschaffen sind, dass sie die Luftundurchlässigkeit zwischen der besagten Muffe und dem Bohrloch (18) gewährleisten.

13. Reinigungsvorrichtung gemäß Patentanspruch 12, **dadurch gekennzeichnet, dass** die äußeren Dichtlippen (22) so beschaffen sind, dass sie sich gegenläufig zur Einschubrichtung der Muffe (20) in das Bohrloch (18) biegen.
